# EUROPEAN PATENT SPECIFICATION

(11) **EP 4 545 971 B1**
(45) Date of publication and mention of the grant of the patent: **28.10.2026**
(21) Application number: 25161091.1
(22) Date of filing: 15.07.2021
(51) Int. Cl.: C07K 7/06, C07K 7/08, A61K 38/00, A61P 31/14, G01N 33/569

(54) **PEPTIDE WITH NEUTRALIZING ACTIVITY AGAINST SEVERE ACUTE RESPIRATORY SYNDROME CORONAVIRUS 2**
PEPTID MIT NEUTRALISIERENDER WIRKUNG GEGEN SCHWERES AKUTES RESPIRATORISCHES SYNDROM-CORONAVIRUS 2
PEPTIDE AYANT UNE ACTIVITÉ NEUTRALISANTE CONTRE LE CORONAVIRUS 2 DU SYNDROME RESPIRATOIRE AIGU SEVERE

(30) Priority: 16.07.2020 KR 20200088076; 09.03.2021 KR 20210030604
(43) Date of publication of application: 30.04.2025
(62) Document number(s) of the earlier application(s) in accordance with Art. 76 EPC: 21842944.7 / 4 183 793
(73) Proprietor: Caregen Co., Ltd., Anyang-si, Gyeonggi-do 14119 (KR)
(72) Inventor: CHUNG, Yong Ji, 14119 Anyang-si, Gyeonggi-do (KR); KIM, Eun Mi, 14119 Anyang-si, Gyeonggi-do (KR); LEE, Eung Ji, 14119 Anyang-si, Gyeonggi-do (KR); LEE, Young Min, 14119 Anyang-si, Gyeonggi-do (KR); KANG, Han A, 14119 Anyang-si, Gyeonggi-do (KR); JEONG, Min Kyeong, 14119 Anyang-si, Gyeonggi-do (KR)
(74) Representative: Regimbeau

(56) References cited:
- KR-A- 20140 134 083
- KR-A- 20170 105 697
- XIA SHUAI ET AL: "Inhibition of SARS-CoV-2 (previously 2019-nCoV) infection by a highly potent pan-coronavirus fusion inhibitor targeting its spike protein that harbors a high capacity to mediate membrane fusion", CELL RESEARCH, SPRINGER SINGAPORE, SINGAPORE, vol. 30, no. 4, 30 March 2020 (2020-03-30), pages 343 - 355, XP037082964, ISSN: 1001-0602, [retrieved on 20200330], DOI: 10.1038/S41422-020-0305-X
- CHANGHAI LEI ET AL: "Neutralization of SARS-CoV-2 spike pseudotyped virus by recombinant ACE2-Ig", NATURE COMMUNICATIONS, 24 April 2020 (2020-04-24), England, pages 1 - 5, XP055745525, Retrieved from the Internet <URL:https://www.nature.com/articles/s41467-020-16048-4.pdf> DOI: 10.1038/s41467-020-16048-4
- ZOST SETH J ET AL: "Rapid isolation and profiling of a diverse panel of human monoclonal antibodies targeting the SARS-CoV-2 spike protein", NATURE MEDICINE, NATURE PUBLISHING GROUP US, NEW YORK, vol. 26, no. 9, 10 July 2020 (2020-07-10), pages 1422 - 1427, XP037241576, ISSN: 1078-8956, [retrieved on 20200710], DOI: 10.1038/S41591-020-0998-X
- ZHANG G. ET AL: "Investigation of ACE2 N-terminal fragments binding to SARS-CoV-2 Spike RBD", BIORXIV, 17 June 2020 (2020-06-17), XP093107462, Retrieved from the Internet <URL:https://www.biorxiv.org/content/10.1101/2020.03.19.999318v2.full.pdf> [retrieved on 20231130], DOI: 10.1101/2020.03.19.999318

## Description

### [Technical Field]

The present invention relates to a peptide having neutralizing activity against severe acute respiratory syndrome coronavirus 2 (SARS-Cov-2) and the use thereof.

### [Background Art]

Severe acute respiratory syndrome coronavirus 2 (SARS-Cov-2) is a single-stranded RNA virus that belongs to the subfamily *Coronavirinae,* and was first discovered in December 2019 in a patient with pneumonia of unknown cause in Wuhan, China. To avoid confusion with severe acute respiratory syndrome infection, the World Health Organization (WHO) also refers to it as "the coronavirus disease 2019 virus" or "the virus responsible for coronavirus disease 2019".

It is known that from December 2019 to early July 2020, 11.57 million confirmed cases occurred in 214 countries around the world, of which 537,138 died, and the fatality rate reaches 4.64. The clinical symptoms of SARS-CoV-2 are mainly respiratory symptoms accompanied by fever, and the main vulnerable subjects are the elderly in their 60s or older suffering from underlying diseases, immune diseases, and the like. Unlike previous viruses, it showed strong contagiousness and rapid worsening of symptoms, and in March 2020, about three months after the first outbreak was reported, the WHO declared a global epidemic, a pandemic.

There is no specific therapeutic agent yet, but it has been reported that research on antiviral agents was conducted, and antiviral drugs such as remdesivir, an RNA polymerase inhibitor, chloroquine, a malaria drug, and lopinavir/ritonavir, an antiretroviral human immunodeficiency I protease inhibitor, showed SARS-CoV-2 inhibitory effects at the cellular level. However, therapeutic agents that have been known to be effective so far have become problematic in actual clinical practice because their effects are insignificant or they can cause various side effects. Some authors disclosed specific peptides (fusion proteins, antibodies) for their ability to inhibit SARS- CoV-2 S. For example: Xia Shuai et al, (2019) discloses a protein, EK1C4, which is a potent fusion inhibitor against SARS- CoV-2 S protein- mediated membrane fusion and pseudovirus infection, the said protein specifically recognizing the spike protein. Changhai Lei et al., (2020) discloses a recombinant protein by connecting the extracellular domain of human ACE2 to the Fc region of the human immunoglobulin IgG1, said protein specifically recognizing the SARS-CoV-2 RBD domain. Zost Seth J et al. (2020) discloses human monoclonal antibodies (mAbs) against the SARS-CoV-2 spike (S) protein. Zhang G. et al. (2020) discloses a SBP1 protein associated with SARS-CoV-2-RBD protein. However, there is still a need of novel more efficient therapeutic agents.

Accordingly, as a result of diligent efforts to develop a new therapeutic agent capable of inhibiting SARS-CoV-2, the present inventors have confirmed that the peptides of the present invention may be usefully used for preventing, ameliorating or treating coronavirus infection, and have completed the present invention.

### [Disclosure]

### [Technical Problem]

It is an object of the present invention to provide a peptide that specifically recognizes a protein of severe acute respiratory syndrome coronavirus 2 (SARS-Cov-2) or a portion thereof.

It is disclosed herewith but not claimed a composition for preventing or treating SARS-CoV-2 infection.

It is disclosed herewith but not claimed a method of preventing or treating SARS-CoV-2 infection.

It is still another object of the present invention to provide a pharmaceutical composition for use in preventing or treating SARS-CoV-2 infection. comprising the peptide of the invention.

It is disclosed but not claimed a composition for detecting SARS-CoV-2.

It is disclosed but not claimed a method of detecting SARS-CoV-2.

It is still another object of the present invention to provide the use of a composition comprising the peptide of the invention for detecting in vitro severe acute respiratory syndrome coronavirus 2 (SARS-CoV-2),

It is disclosed but not claimed a kit for diagnosing SARS-CoV-2 infection or disease.

### [Technical Solution]

To these ends, an aspect of the present invention provides a peptide that specifically recognizes a protein of severe acute respiratory syndrome coronavirus 2 (SARS-Cov-2) or a portion thereof consisting of an amino acid sequence of SEQ ID NO: 2.

In addition, it is disclosed but not claimed a composition for preventing or treating SARS-CoV-2 infection, comprising the peptide.

In addition, it is disclosed but not claimed a method of preventing or treating SARS-CoV-2 infection, comprising administering the peptide or the composition comprising the peptide to a subject.

In addition, another aspect of the present invention provides a pharmaceutical composition comprising the peptide for use in preventing or treating SARS-CoV-2 infection.

In addition, it is disclosed but not claimed a composition for detecting SARS-CoV-2, comprising the peptide.

In addition, it is disclosed but not claimed a method of detecting SARS-CoV-2, comprising contacting the peptide or the composition comprising the peptide with a biological sample.

In addition, another aspect of the present invention provides the use of a of a composition comprising the peptide of the invention for detecting in vitro severe acute respiratory syndrome coronavirus 2 (SARS-CoV-2).

Another aspect it is disclosed but not claimed a kit for diagnosing SARS-CoV-2 infection or disease.

### [Advantageous Effects]

The peptide consisting of the amino acid sequences of SEQ ID NO: 2 according to the present invention has the effect of inhibiting the membrane fusion of a virus and a host cell by binding to a spike protein on the surface of SARS-CoV-2 to neutralize SARS-CoV-2, and thus, it may be used for the purpose of preventing, treating or diagnosing SARS-CoV-2

### [Description of Drawings]

Figure 1 is graphs showing the ELISA results of confirming the ability of the peptides of SEQ ID NOs: 1 to 6 to bind to the receptor binding domain (RBD) of the SARS-CoV-2 spike protein.
Figure 2 is graphs showing the ELISA results of confirming the activity of the peptides of SEQ ID NOs: 1 to 6 to inhibit the binding of the SARS-CoV-2 spike protein RBD to the angiotensin-converting enzyme 2 (ACE2) protein of the host cell.
Figure 3 shows the Western blot results of confirming whether the peptides of SEQ ID NOs: 1 to 4 have the ability to inhibit the binding of RBD to ACE2 expressed on the cell surface of human lung cancer cell line A549.

### [Best Mode]

Hereinafter, the present invention will be described in detail.

An aspect of the present invention provides a peptide that specifically recognizes a protein of severe acute respiratory syndrome coronavirus 2 (SARS-CoV-2) or a portion thereof, consisting of sequences of SEQ ID NO: 2.

In the present specification, the term "peptide" refers to a linear or cyclic molecule formed by linking amino acid residues to each other by a peptide bond. The preparation of the peptide may be achieved by a conventional biological or chemical synthesis method known in the art, and as an example, it may be achieved by methods such as solid-phase synthesis techniques.

The "peptide" may be variants or fragments of amino acids having a different sequence by deletion, insertion or substitution of amino acid residues, or a combination thereof within a range that does not affect the function. Amino acid exchanges that do not entirely alter the activity of the peptide are known in the art. In some cases, it may be modified by phosphorylation, sulfation, acrylation, glycosylation, methylation, farnesylation, and the like. Accordingly, the present invention includes a peptide consisting of SEQ ID NOs: 2. In addition, the peptide of the present invention may further comprises a targeting sequence, a tag, a labeled residue, and an amino acid sequence prepared for a specific purpose of increasing half-life or peptide stability.

In addition, in order to obtain better chemical stability, enhanced pharmacological properties (half-life, uptake, titer, efficacy, and the like), altered specificity (for example, a broad spectrum of biological activity), and reduced antigenicity, a protecting group may be attached to the N-terminus or C-terminus of the peptides of the present invention. For example, the protecting group may be an acetyl group, a fluorenylmethoxycarbonyl group, a formyl group, a palmitoyl group, a myristyl group, a stearyl group, or polyethylene glycol (PEG), but any component capable of enhancing the modification of the peptide, in particular, the stability of the peptide may be included without limitation. The "stability" is used as a meaning including not only *in vivo* stability that protects the peptides of the present invention from attack by *in vivo* protein cleaving enzymes, but also storage stability (for example, storage stability at room temperature). The peptide of the present invention consists of : KFNRRHH (SEQ ID NO: 2). It is also described herewith but not claimed peptides consisting of any of the sequences selected in the group of:
RSYMTTHHEQF (SEQ ID NO: 1);
KYLLVHRPYYRR (SEQ ID NO: 3);
WVPYQARVPYPR (SEQ ID NO: 4);
RLYCKNGGFFLR (SEQ ID NO: 5); and
KHRGGGNRR (SEQ ID NO: 6).

The peptides may specifically bind to the spike protein (S protein) of SARS-CoV-2. More specifically, the peptides may specifically bind to the RBD (receptor binding domain) present in the spike protein of SARS-CoV-2.

In the present specification, the term "specifically binding" means binding to the protein of interest, i.e., the RBD that is a spike protein of SARS-CoV-2 or a portion thereof, but not substantially recognizing and binding to other molecules in the sample.

The peptides may specifically bind to a spike protein on the surface of SARS-CoV-2 to inhibit the binding of the spike protein to the cell membrane receptor of a host cell.

The cell membrane receptor of the host cell is angiotensinconvering enzyme 2 (ACE2), and the angiotensin converting enzyme 2 is a transmembrane protein found in eukaryotes and bacteria and is a receptor used by coronaviruses including SARS-CoV-2 to invade cells.

In an embodiment, it was confirmed that the peptides of the present invention may specifically bind to the spike protein RBD on the surface of SARS-CoV-2 to neutralize SARS-CoV-2, and may inhibit the binding of SARS-CoV-2 to the cell membrane of the host cell.

In the present specification, the term "neutralization" refers to the ability to inhibit the replication of SARS-CoV-2 *in vivo* and/or *in vitro,* regardless of the mechanism by which neutralization is achieved. In an embodiment, the peptides of the present invention neutralize SARS-CoV-2 by inhibiting SARS-CoV-2 from attaching to target cells to fuse the viral membrane with the cell membrane.

It is also described a pharmaceutical composition for preventing or treating severe acute respiratory syndrome coronavirus 2 (SARS-CoV-2) infection, comprising the peptide.

In addition, it is disclosed a method of preventing or treating SARS-CoV-2 infection, comprising administering the peptide or the composition comprising the peptide to a subject.

In addition, the present invention provides a pharmaceutical composition comprising the peptide for use in preventing or treating SARS-CoV-2 infection. The composition may prevent or treat SARS-CoV-2 infection by binding to a spike protein on the surface of severe acute respiratory syndrome coronavirus 2 to inhibit the binding of the spike protein to a cell membrane receptor of a host cell.

The pharmaceutical composition for preventing or treating SARS-CoV-2 infection according to the present invention may be formulated and used in the form of oral formulations such as powders, granules, tablets, capsules, suspensions, emulsions, syrups and aerosols, external preparations, suppositories and sterile injectable solutions according to conventional methods, respectively, and may further comprise a pharmaceutically acceptable carrier, excipient or diluent in addition to the peptide for formulation.

The pharmaceutically acceptable carriers may include lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methylcellulose, methylhydroxybenzoate, propylhydroxybenzoate, talc, magnesium stearate, mineral oil, and the like, which are commonly used in the formulations.

The pharmaceutical composition may further comprise a lubricant, a wetting agent, a sweetener, a flavoring agent, an emulsifier, a suspending agent, a preservative, and the like, in addition to the ingredients as described above.

The pharmaceutical composition may be administered orally or parenterally (for example, applied intramuscularly, intravenously, intraperitoneally, subcutaneously, intradermally, or locally) depending on the desired method, and the dosage varies depending on the condition and body weight of the patient, the severity of the disease, the form of the drug, the route and time of administration, but can be appropriately selected by those skilled in the art.

The pharmaceutical composition of the present invention is administered in a pharmaceutically effective amount. In the present invention, the term "pharmaceutically effective amount" refers to an amount sufficient to treat a disease at a reasonable benefit/risk ratio applicable to medical treatment, and the level of the effective dose may be determined depending on the factors including the type and severity of the patient's disease, the activity of the drug, the sensitivity to the drug, the time of administration, the route of administration and the rate of the excretion, the duration of treatment, and the drugs used simultaneously, and other factors well known in the medical field. The pharmaceutical composition may be administered as an individual therapeutic agent or administered in combination with other therapeutic agents, may be administered concurrently, separately, or sequentially with conventional therapeutic agents, and may be administered single or multiple times. Considering all of the above factors, it is important to administer an amount that can achieve the maximum effect in a minimal amount without side effects, which can be easily determined by those skilled in the art.

The effective amount of the pharmaceutical composition may vary depending on the age, sex, condition and body weight of the patient, the degree of absorption of the active ingredient into the body, the rate of the inactivation, the rate of the excretion, the type of disease, and concomitant drugs, and may be increased or decreased depending on the route of administration, the severity of obesity, sex, body weight, age, and the like, and, for example, the pharmaceutical composition may be administered in an amount of about 0.0001 µg to 500 mg, preferably 0.01 µg to 100 mg per 1 kg of patient's body weight per day.

The pharmaceutical composition of the present invention may further comprise at least one other therapeutic agent. For example, it may further comprise interferons, anti-S protein monoclonal antibodies, anti-S protein polyclonal antibodies, nucleoside analogs, DNA polymerase inhibitors, siRNA preparations or therapeutic vaccines as antiviral drugs together with the peptide. These may be used in combination with the peptides of the present invention. In the present specification, "in combination with" refers to following a regimen administered either simultaneously as separate formulations or as one single combined formulation, or sequentially in any order as separate formulations.

The "subject" may be a subject in need of administration of the peptide of the present application or a composition comprising the peptide, and the subject in need of the administration may include a subject diagnosed with SARS-CoV-2 infection, a subject with developed symptoms of SARS-CoV-2 infection, as well as a subject wishing to be administered to prevent the development of the disease or symptom or to improve health.

The "administration" refers to providing a predetermined substance to a patient by any appropriate method, and the peptides of the present invention may be administered orally or parenterally through all general routes as long as it can reach a desired tissue. In addition, the peptide or the composition comprising the peptide may be administered by any device capable of transporting an active substance to a target cell.

In another aspect, it is also disclosed a pharmaceutical composition for detecting severe acute respiratory syndrome coronavirus 2 (SARS-CoV-2), comprising the peptide.

In addition, it is disclosed a method of detecting SARS-CoV-2, comprising contacting the peptide or the composition comprising the peptide with a biological sample.

In addition, the present invention provides the use of a composition comprising the peptide of the invention for detecting in vitro severe acute respiratory syndrome coronavirus 2 (SARS-CoV-2)

The composition for detecting SARS-CoV-2 may be used to detect the presence or absence of SARS-CoV-2 in a biological sample by confirming the reaction after contacting the peptide with the biological sample.

The peptides may be labeled to be detectable. Examples of labels that can be used in the present invention include enzymes, radioisotopes, colloidal metals, fluorescent compounds, chemiluminescent compounds, and bioluminescent compounds. Commonly used labels include fluorescent substances (for example, fluorescein, rhodamine, Texas red, and the like), enzymes (for example, horseradish peroxidase, β-galactosidase, alkaline phosphatase), radioisotopes (for example, 32P or 125I), biotin, digoxigenin, colloidal metals, chemiluminescent or bioluminescent compounds (for example, dioxetane, luminol or acridinium). Labeling methods such as covalent bonding of enzymes or biotinyl groups, iodination, phosphorylation and biotinylation are well known in the art.

Detection of the presence of SARS-CoV-2 may be performed by any one selected from the group consisting of enzyme-linked immunosorbent assay (ELISA), Western blotting, immunofluorescence, immunohistochemistry staining, flow cytometry, immunocytochemical assay, radioimmunoassay (RIA), immunoprecipitation assay, immunodiffusion assay, complement fixation assay, and protein chip.

The enzyme-linked immunosorbent assay (ELISA) includes various ELISA methods such as direct ELISA using a labeled antibody recognizing the peptide attached to a solid support, and indirect ELISA using a labeled secondary antibody recognizing the capture antibody in a complex of antibodies recognizing the peptide attached to a solid support.

The biological sample may be any one selected from the group consisting of, but is not limited to, sputum, spit, blood, sweat, respiratory tissue and saliva of a subject, and the sample may be prepared by a conventional method known in the art.

The present invention also discloses a kit for diagnosing SARS-CoV-2 infection or disease, comprising the peptide.

In the present specification, the term "SARS-CoV-2 infection or disease" refers to a pathological condition resulting from infection of a cell or subject by SARS-CoV-2, and, for example, the disease may be a respiratory disease caused by SARS-CoV-2.

The kit may comprise a solid carrier. The peptides may be attached to the solid carrier, and such a solid carrier may be porous or non-porous, planar or non-planar.

### [Best Mode]

Hereinafter, the present invention will be described in detail by way of examples and experimental examples.

### [Example 1]

### Selection of peptides

In order to develop a peptide that inhibits the binding of a spike protein (S protein) of SARS-CoV-2 to angiotensin-converting enzyme 2 (ACE2), six peptides were selected from the peptide libraries through screening. The sequences of the selected peptides and the molecular weights of the peptides measured using a molecular weight meter are shown in Table 1 below.

**[Table 1]**

| No. | Sequence | Molecular weight (Da) | SEQ ID NO: |
|---|---|---|---|
| 1 | RSYMTTHHEQF | 1437 | 1 |
| 2 | KFNRRHH | 994.1 | 2 |
| 3 | KYLLVHRPYYRR | 1664 | 3 |
| 4 | WVPYQARVPYPR | 1532 | 4 |
| 5 | RLYCKNGGFFLR | 1474 | 5 |
| 6 | KHRGGGNRR | 1037 | 6 |

### [Example 2]

### Confirmation of the ability of said peptides to bind to the SARS-CoV-2 spike protein RBD

In order to confirm whether the peptides may block the invasion of SARS-CoV-2 into host cells, first, it was confirmed whether the peptides may recognize and bind to RBD in the spike protein of SARS-CoV-2.

Specifically, the six peptides selected in Experimental Example 1 were mixed with 2 mM sodium bicarbonate in equal volume and dispensed in 100 µL each in a 96-well plate, and then cultured overnight at 4°C to coat the surface of the well with the peptides. They were washed three times with 0.1% PBS-T (0.1% Tween-20 in PBS), and then 100 µL of 3% BSA was dispensed, and then blocked at room temperature for 1 hour and washed three times with 0.1% PBS-T. Thereafter, they were treated with 100 ng of recombinant SARS-CoV-2 spike RBD-His (Sino Biological, Cat. No.: 40592-V08B, China) per well and reacted at room temperature for 2 hours. They were washed three times with 0.1% PBS-T, and then treated with anti-His antibody-HRP (Abcam, Cat. No.: ab1187, US) diluted 1:1000 in PBS and reacted at room temperature for 1 hour, and then washed three times with 0.1% PBS-T. 100 µL of TMB solution (ThermoFisher, Cat. No.: N301, US) was dispensed and reacted for about 5 minutes, and then the reaction was terminated by treatment with 50 µL of 1 M sulfuric acid. O.D. values were measured at a wavelength of 450 nm using a spectrometer.

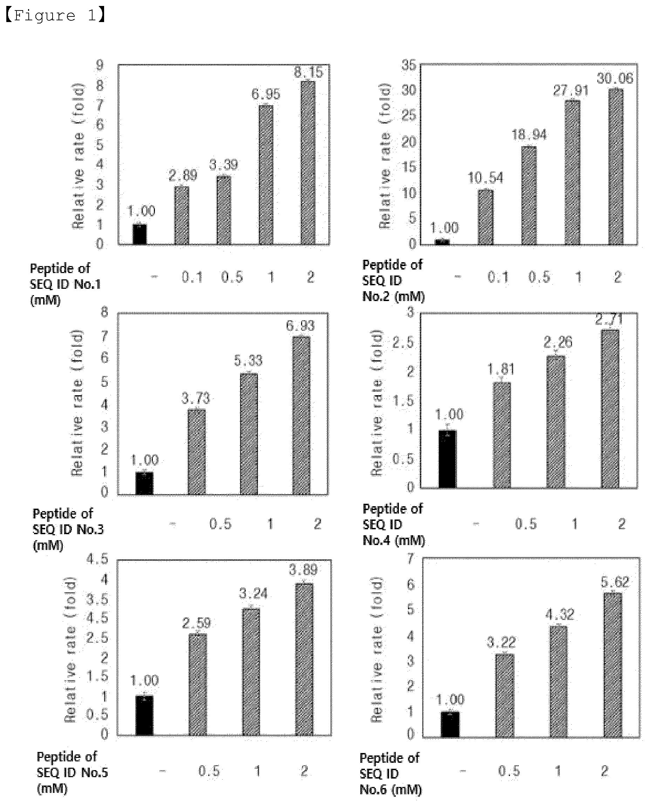

As a result, as shown in Figure 1, when the binding pattern of each of the peptides of SEQ ID NOs: 1 to 6 to RBD was observed by ELISA, it was confirmed that the binding fold of the peptides to RBD increased in a concentration-dependent manner.

From the above, it can be seen that the peptides of the present invention have the ability to recognize and bind to the RBD of the SARS-CoV-2 spike protein.

### [Example 3]

### Confirmation of the ability of said peptides to inhibit ACE2-RBD binding

In order to confirm whether the peptides may block the invasion of SARS-CoV-2 into host cells, it was confirmed whether the peptides of the present invention inhibit the binding of ACE2, which is a membrane protein of host cells, to the SARS-CoV-2 RBD.

Specifically, 2 mM sodium bicarbonate and ACE2-FC protein (Sino Biological, Cat. No.: 10108-H02H, China) were mixed in equal volume and dispensed in 100 ng/100 µL/well in a 96-well plate, an then cultured overnight at 4°C to coat the surface of the well with ACE2 protein. They were washed three times with 0.1% PBS-T (0.1% Tween-20 in PBS), and then 100 µL of 3% BSA was dispensed, and then blocked at room temperature for 1 hour. 100 ng of recombinant SARS-CoV-2 spike RBD His (Sino Biological, Cat. No.: 40592-V08B, China) was mixed with a peptide solution at each concentration in PBS and reacted at room temperature for 1 hour. The wells were washed 3 times with 0.1% PBS-T, and then the wells were treated with the protein-peptide mixture. They were reacted at room temperature for 2 hours, and then washed three times with 0.1% PBS-T. They were treated with anti-His antibody-HRP (Abcam, Cat. No.: ab1187, US) diluted 1:1000 in PBS and reacted at room temperature for 1 hour, and then washed three times with 0.1% PBS-T. 100 µL of TMB solution (ThermoFisher, Cat. No.: N301, US) was dispensed and reacted for about 5 minutes, and then the reaction was terminated by treatment with 50 µL of 1 M sulfuric acid. O.D. values were measured at a wavelength of 450 nm using a spectrometer.

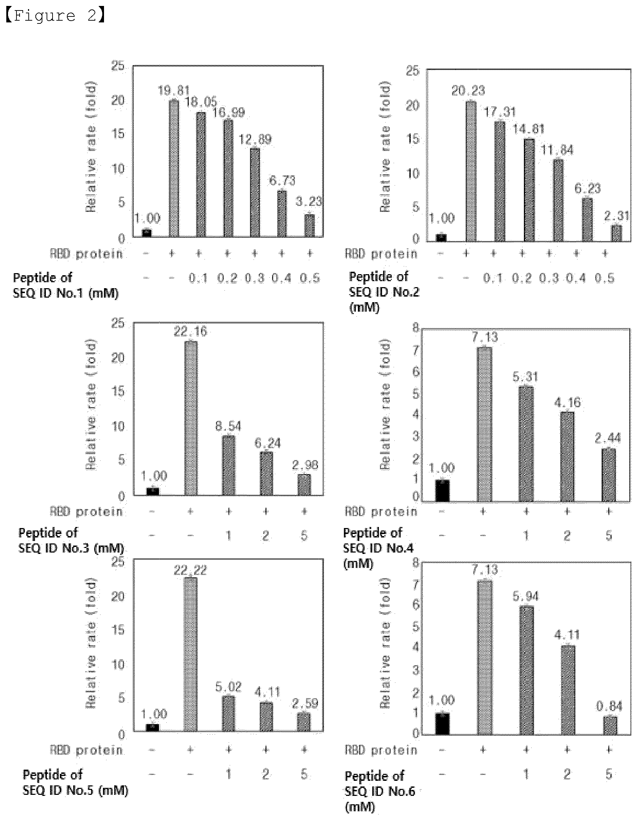

As a result, as shown in Figure 2, it was confirmed that each of the peptides of SEQ ID NOs: 1 to 6 inhibited the binding of RBD to the ACE2 protein in a concentration-dependent manner.

From the above, it can be seen that the peptides bind to the RBD of the SARS-CoV-2 spike protein to inhibit the binding of the spike protein to the ACE2 protein.

### [Example 4]

### Confirmation of the ability of said peptides to inhibit ACE2-RBD binding in human lung cancer cells

In order to confirm whether the peptides have the ability to prevent, ameliorate and treat coronavirus infection in mammalian cells, it was confirmed whether the ability of the SARS-CoV-2 spike protein RBD to bind to ACE2 of host cells is reduced after a human lung cancer cell line were treated with the peptides.

Specifically, A549 cells (Korean Cell Line Bank, Cat. No.: 10185, Korea), which are a human lung cancer cell line (adenocarcinomic human alveolar basal epithelial cells), were inoculated in a 6-well plate at a density of 5x10⁵ cells/well and cultured for 24 hours. 500 ng of recombinant SARS-CoV-2 spike RBD His (Sino Biological, Cat. No.: 40592-V08B, China) was mixed with a peptide solution at each concentration in 1 mL of DMEM medium and reacted at room temperature for 1 hour. A549 cells cultured for one day were washed with DMEM medium, and then 900 µL of recombinant SARS-CoV-2 spike RBD/peptide mixture was added to the cells. After culturing for 2 hours in a CO₂ incubator at 37°C, the cells were washed twice with PBS and treated with 200 µL of lysis buffer to lyse the cells. Samples were prepared by treatment with 5X sample buffer, and then SDS-PAGE was performed using a 10% SDS-PAGE gel. Proteins separated through SDS-PAGE were transferred to a PVDF membrane, and then blocked using 5% skim milk at room temperature for 1 hour. Next, anti-His antibody-HRP (Abcam, Cat. No.: ab1187, US) was diluted 1:1000 in 5% skim milk, reacted with the membrane for 2 hours, and washed three times with 0.1% PBS-T (0.1% Tween-20 in PBS) for 10 minutes. It was detected as a film using an ECL solution (GE Healthcare, Cat. No.: RPN2232, US).

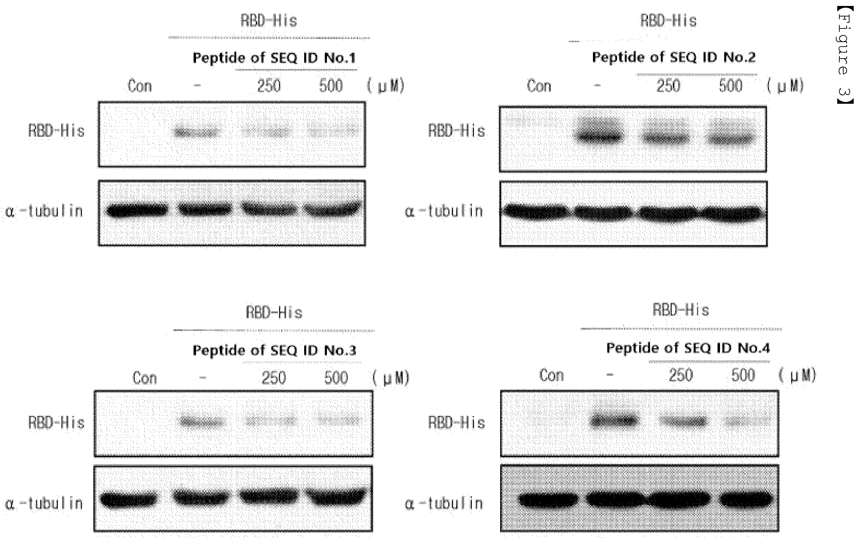

As a result, as shown in Figure 3, it was confirmed that each of the peptides of SEQ ID NOs: 1 to 4 inhibited the binding of the SARS-CoV-2 spike RBD to ACE2 of host cells in a concentration-dependent manner.

From the above, it can be seen that the peptides may prevent or treat virus infection in host cells by binding to the spike protein of SARS-CoV-2 to inhibit the binding of the spike protein to ACE2 of host cells.

### [Example 5]

### Confirmation of the effect of said peptides to inhibit SARS-CoV-2 cell infection

In order to directly confirm whether the peptides have the effect of inhibiting cell infection by SARS-CoV-2, a focus reduction neutralizing test (FRNT) was performed.

Specifically, Vero E6 cells (ATCC, USA) were inoculated in a 96-well plate at a density of 2x10⁴ cells/well, and then cultured until 90-100% confluency. 500 focus-forming units (FFU)/well of SARS-CoV-2 and peptide samples at each concentration were mixed in a 1:1 ratio, and reacted at 37°C for 1 hour. A monolayer of Vero E6 cells was treated with the mixture of peptide and virus, and culture at 37°C for 1 hour. The supernatant was removed, and then treated with DMEM medium containing 1.6% carboxymethylcellulose (CMC) and cultured at 37°C for 24 hours. The supernatant was removed, and then the cells were fixed with 4% paraformaldehyde and washed with PBS. Cell permeabilization was performed for 30 minutes by treatment with PBS containing 0.2% Triton X-100 and 1% BSA, and then washed with PBS. The cells were treated for 1 hour with the primary antibody against SARS-CoV-2 nucleocapsid (Sino Biological, China) diluted 1:1000 in PBS containing 1% BSA, and washed with PBST (PBS containing 1% Tween-20). The cells were treated for 1 hour with the secondary antibody (Sino Biological, China) diluted 1:2000 in PBS containing 1% BSA, and washed with PBST. They were treated with KPL TrueBlue^{™} Peroxidase Substrate (SeraCare Life Sciences, USA), and then washed with distilled water, and the number of SARS-CoV-2 foci was analyzed by Elispot reader (Cellular Technology Limited, USA). The inhibition rate against viral infection was calculated using the following equation:

### Equation 1

Inhibition rate against virus infection (%) = (number of foci in the control group - number of foci in the treated group)/number of foci in the control group X 100

As a result, as shown in Table 2, it was confirmed that when treated with the peptides of SEQ ID NOs: 1 to 4 according to the present invention, the inhibition rate against viral infection increased in a concentration-dependent manner for all four peptides.

From the above, it can be seen that the peptides may prevent, ameliorate and treat SARS-CoV-2 infection by inhibiting cell infection by SARS-CoV-2.

**[Table 2]**

| **SEQ ID NO:** | **Concentration (mM)** | **Inhibition rate (%)** |
|---|---|---|
| 1 | 2.5 | 21 |
| | 10 | 100 |
| 2 | 2.5 | 51 |
| | 10 | 96 |
| 3 | 2.5 | 98 |
| | 10 | 98 |
| 4 | 2.5 | 55 |
| | 10 | 98 |

## Claims

1. A peptide that specifically recognizes a protein of severe acute respiratory syndrome coronavirus 2 (SARS-CoV-2) or a portion thereof, consisting of an amino acid sequence of SEQ ID NO: 2.

2. The peptide according to claim 1, wherein the protein of severe acute respiratory syndrome coronavirus 2 is a spike protein.

3. The peptide according to claim 1, wherein the peptide binds to a spike protein on the surface of severe acute respiratory syndrome coronavirus 2 to inhibit the binding of the spike protein to a cell membrane receptor of a host cell.

4. A pharmaceutical composition comprising the peptide of claim 1.

5. A pharmaceutical composition comprising the peptide of claim 1 for use in preventing or treating severe acute respiratory syndrome coronavirus 2 (SARS-CoV-2) infection.

6. The pharmaceutical composition for use according to claim 5, wherein the composition binds to a spike protein on the surface of severe acute respiratory syndrome coronavirus 2 to inhibit the binding of the spike protein to a cell membrane receptor of a host cell.

7. The pharmaceutical composition for use according to claim 5, wherein the pharmaceutical composition further comprises a pharmaceutically acceptable carrier, excipient or diluent.

8. The pharmaceutical composition for use according to claim 5, wherein the pharmaceutical composition is formulated in the form of oral preparations, injection preparations or external preparations.

9. Use of a composition comprising the peptide of claim 1 for detecting in vitro severe acute respiratory syndrome coronavirus 2 (SARS-CoV-2).

## Patentansprüche

1. Peptid, das spezifisch ein Protein von Coronavirus 2 des schweren akuten respiratorischen Syndroms (SARS-CoV-2) oder einen Abschnitt davon erkennt, das aus einer Aminosäuresequenz mit der SEQ ID NR.: 2 besteht.

2. Peptid nach Anspruch 1, wobei das Protein von Coronavirus 2 des schweren akuten respiratorischen Syndroms ein Spike-Protein ist.

3. Peptid nach Anspruch 1, wobei das Peptid sich an ein Spike-Protein auf der Oberfläche von Coronavirus 2 des schweren akuten respiratorischen Syndroms bindet, um das Binden des Spike-Proteins an einen Zellmembran-Rezeptor einer Wirtszelle zu hemmen.

4. Pharmazeutische Zusammensetzung, die das Peptid nach Anspruch 1 umfasst.

5. Pharmazeutische Zusammensetzung, die das Peptid nach Anspruch 1 umfasst, zur Verwendung bei der Prävention oder Behandlung einer Infektion mit Coronavirus 2 des schweren akuten respiratorischen Syndroms (SARS-CoV-2).

6. Pharmazeutische Zusammensetzung zur Verwendung nach Anspruch 5, wobei die Zusammensetzung sich an ein Spike-Protein auf der Oberfläche von Coronavirus 2 des schweren akuten respiratorischen Syndroms bindet, um das Binden des Spike-Proteins an einen Zellmembran-Rezeptor einer Wirtszelle zu hemmen.

7. Pharmazeutische Zusammensetzung nach Anspruch 5, wobei die pharmazeutische Zusammensetzung ferner einen pharmazeutisch verträglichen Trägerstoff, Hilfsstoff oder ein pharmazeutisch verträgliches Verdünnungsmittel umfasst.

8. Pharmazeutische Zusammensetzung zur Verwendung nach Anspruch 5, wobei die pharmazeutische Zusammensetzung in der Form von oralen Präparaten, Injektionspräparaten oder Präparaten zur äußerlichen Anwendung formuliert ist.

9. Verwendung einer Zusammensetzung, die das Peptid nach Anspruch 1 umfasst, zum *In-vitro*-Nachweisen von Coronavirus 2 des schweren akuten respiratorischen Syndroms (SARS-CoV-2).

## Revendications

1. Peptide reconnaissant spécifiquement une protéine du coronavirus 2 du syndrome respiratoire aigu sévère (SARS-CoV-2) ou une partie de celle-ci, constitué de la séquence d'acides aminés de SEQ ID NO : 2.

2. Peptide selon la revendication 1, dans lequel la protéine du coronavirus 2 du syndrome respiratoire aigu sévère est une protéine Spike.

3. Peptide selon la revendication 1, dans lequel le peptide se lie à une protéine Spike à la surface du coronavirus 2 du syndrome respiratoire aigu sévère afin d'inhiber la liaison de la protéine Spike à un récepteur membranaire d'une cellule hôte.

4. Composition pharmaceutique comprenant le peptide selon la revendication 1.

5. Composition pharmaceutique comprenant le peptide selon la revendication 1, destinée à être utilisée pour prévenir ou traiter une infection par le coronavirus 2 du syndrome respiratoire aigu sévère (SARS-CoV-2).

6. Composition pharmaceutique destinée à être utilisée selon la revendication 5, dans laquelle la composition se lie à une protéine Spike présente à la surface du coronavirus 2 du syndrome respiratoire aigu sévère afin d'inhiber la liaison de ladite protéine Spike à un récepteur membranaire d'une cellule hôte.

7. Composition pharmaceutique destinée à l'usage selon la revendication 5, dans laquelle la composition pharmaceutique comprend en outre un support, un excipient ou un diluant pharmaceutiquement acceptable.

8. Composition pharmaceutique destinée à être utilisée selon la revendication 5, dans laquelle la composition pharmaceutique est formulée sous forme de préparations orales, de préparations injectables ou de préparations à usage externe.

9. Utilisation d'une composition comprenant le peptide de la revendication 1 pour la détection in vitro du coronavirus 2 du syndrome respiratoire aigu sévère (SARS-CoV-2).
